# FASCICULE DE BREVET EUROPEEN

(11) **EP 3 173 126 B1**
(45) Date de publication et mention de la délivrance du brevet: **25.03.2020**
(21) Numéro de dépôt: 16200249.7
(22) Date de dépôt: 23.11.2016
(51) Int. Cl.: A61N 1/375, A61N 1/372, A61B 5/00, A61B 5/042, A61B 5/07, A61N 1/05

(54) **CAPSULE IMPLANTABLE, NOTAMMENT CAPSULE AUTONOME DE STIMULATION CARDIAQUE, ET SON PROCEDE D'ASSEMBLAGE**
IMPLANTIERBARE KAPSEL, INSBESONDERE AUTONOME KAPSEL ZUR STIMULATION DER HERZFUNKTION, UND ENTSPRECHENDES MONTAGEVERFAHREN
IMPLANTABLE CAPSULE, IN PARTICULAR AN AUTONOMOUS CARDIAC STIMULATION CAPSULE, AND ITS METHOD OF ASSEMBLY

(30) Priorité: 27.11.2015 FR 1561457
(43) Date de publication de la demande: 31.05.2017
(73) Titulaire: Sorin CRM SAS, 92140 Clamart (FR)
(72) Inventeur: REGNIER, Willy, 91160 LONGJUMEAU (FR)
(74) Mandataire: Grünecker Patent- und Rechtsanwälte PartG mbB

(56) Documents cités:
- US-A1- 2012 116 489
- US-A1- 2012 158 111
- US-A1- 2012 172 690
- US-A1- 2012 172 892
- US-A1- 2014 058 240
- US-A1- 2014 330 326

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au cœur des impulsions électriques de stimulation, de resynchronisation et/ou de défibrillation en cas de trouble du rythme détecté par le dispositif.

L'invention concerne notamment, mais de manière non limitative, ceux de ces dispositifs qui se présentent sous forme d'une capsule autonome destinée à être implantée dans une cavité cardiaque (ventricule ou oreillette, à droite ou à gauche). Ces capsules sont dépourvues de toute liaison mécanique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless",* pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

Deux catégories de capsules autonomes existent. La première catégorie concerne les capsules endocavitaires, qui sont placées dans une des cavités cardiaques. La seconde catégorie concerne les capsules épicardiques qui sont fixées sur la paroi externe du myocarde aussi appelée épicarde.

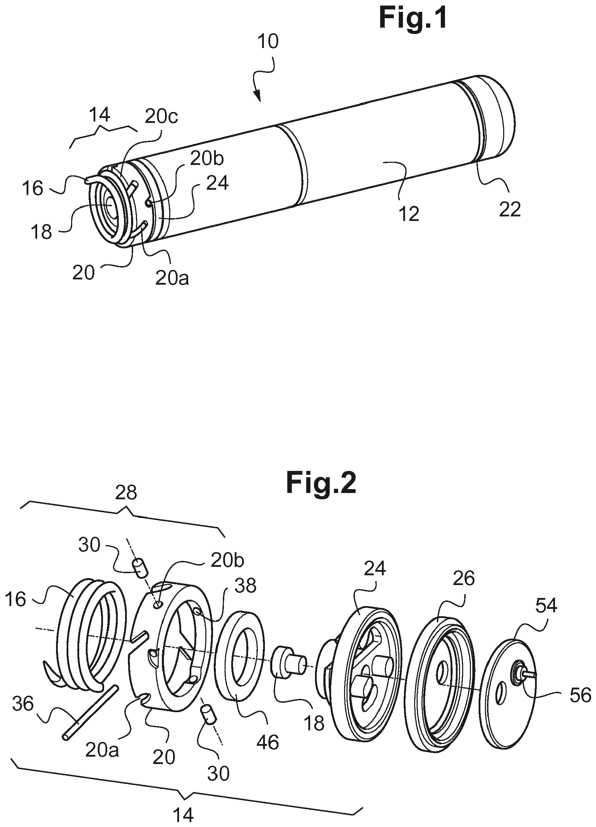

Les capsules endocavitaires ont une forme cylindrique, par exemple de gélule ou de capsule tel qu'illustré en Figure 1 afin de pouvoir être introduites de manière longitudinale par un accessoire d'implantation *in situ,* tel qu'un cathéter à partir du système veineux ou artériel du patient.

À l'extrémité de la capsule se trouve un moyen de fixation afin d'ancrer la capsule au site de stimulation souhaité.

Une capsule implantable telle que décrite dans le US 2008/088397 A1 comprend un corps abritant les principaux éléments du dispositif (circuits électroniques, source d'énergie, électrodes de stimulation, etc.) et une embase solidaire du corps et supportant rigidement un moyen de fixation à la paroi, notamment à la paroi endocavitaire.

Dans le cas des sondes cardiaques, deux types de fixation sont reconnus et traditionnellement employés : la fixation dite à barbes est la plus ancienne et encore marginalement utilisée, mais les sondes basées sur une vis de fixation ont supplanté les sondes à barbes et représentent actuellement la majorité du marché. Elles permettent une fixation généralement robuste et efficace. La vis est une vis hélicoïdale saillante prolongeant axialement le corps de la capsule et destinée à pénétrer dans le tissu cardiaque par vissage au site d'implantation, de la même manière que pour les sondes à vis conventionnelles.

Toutefois, la fixation de tels dispositifs reste un point critique dans la mesure où un détachement accidentel de la capsule amènerait cette dernière à être libérée dans la cavité cardiaque puis transportée par le sang dans le système veineux ou artériel. Le risque de complication pour le patient serait alors extrêmement élevé, ainsi que le risque de blessure du système cardiaque qui peut être engendré par l'extrémité du système de fixation ou toutes autres zones saillantes de l'implant comme par exemple une électrode pointue ou une arête saillante.

Davantage qu'un dispositif à sonde, un dispositif autonome subit quant à lui les contraintes et les mouvements engendrés par la paroi cardiaque, car il ne bénéficie pas de l'effort axial de maintien de la part du corps de sonde. De plus, il présente une certaine masse.

Pour remplir sa fonction d'ancrage permanent, le système de fixation doit donc comporter également une fonction d'irréversibilité, c'est-à-dire qu'il ne pourra être retiré de la paroi cardiaque que par l'intervention volontaire du médecin et suivant une procédure prédéfinie, mais en aucun cas par les mouvements répétés du cœur.

De plus, le médecin doit pouvoir positionner et implanter la capsule à un emplacement choisi par lui, mais aussi pouvoir repositionner la capsule à un autre emplacement si le premier emplacement ne permet pas d'atteindre les performances attendues.

Ainsi, le système d'implantation de la capsule doit être simple et intuitif pour le médecin, notamment en adoptant des procédures d'implantation proche de la pratique courante, qui fasse appel à des gestes bien connus et maitrisés des praticiens, notamment pour l'implantation des sondes cardiaques.

L'utilisation d'un moyen de fixation comprenant un système de vis d'ancrage est notamment décrite dans le document EP 2 818 201 (SORIN CRM SAS) et un accessoire d'implantation *in situ* d'une telle capsule est décrit dans le document EP 2 818 202 (SORIN CRM SAS).

En outre, ces capsules comprennent un dispositif de communication permettant de communiquer avec un dispositif externe, par exemple un programmateur, par radiofréquence ou par le corps humain (HBC) ou tout autre système, et également avec un ou plusieurs autres implants, pour la transmission et la réception d'information.

Afin, pour la capsule, de transmettre et de recevoir des informations cohérentes, il est nécessaire que l'environnement de communication ne soit pas perturbé, notamment par des champs électriques ou autre. En outre pour maintenir une durée de vie de la capsule acceptable après implantation, c'est-à-dire environ 10 ans, le coût énergétique nécessaire à la communication avec un dispositif externe doit être aussi petit que possible.

En effet, le coût énergétique engendré par cette fonction de communication n'est pas négligeable et doit être réduit au minimum pour garantir une longévité maximum de l'implant. Le bilan de liaison entre un implant et le ou les périphériques est prépondérant pour garantir l'échange de données et implique d'avoir un champ électrique le moins perturbé possible et donc un bilan de liaison avec une atténuation la plus faible possible.

Toutefois, il a été observé que la proximité du moyen de fixation métalliques et des électrodes de stimulation provoque la formation d'un rayonnement de champ électrique, notamment entre d'une part, le moyen de fixation et une première électrode et d'autre part, la seconde électrode. Les champs électriques ainsi créés viennent perturber la communication entre la capsule et le dispositif externe de sorte à potentiellement corrompre les informations transmises entre les deux dispositifs.

Une solution connue pour réduire la formation de champs électriques consiste à recouvrir les surfaces externes du moyen de fixation avec un revêtement isolant, par exemple du parylène. Toutefois, un tel revêtement d'une épaisseur de 10 microns environ ne permet pas de résister à une usure liée aux mouvements mécaniques du cœur. En effet, durant la durée de vie de la capsule, à savoir environ 10 ans, la capsule sera soumise à environ 400 millions de cycles cardiaques. L'adhésion du revêtement sur le moyen de fixation ne peut résister à de telles contraintes mécaniques. De plus, des fissures ou des craqures du revêtement peuvent également être causées par de micromouvements de la vis de fixation, notamment du fait du mouvement de la paroi cardiaque.

En outre, il est très difficile d'isoler l'extrémité du moyen de fixation, cette extrémité étant en général pointue. Une mauvaise isolation de cette extrémité entraine la génération de courant de fuite.

De plus, le moyen de fixation comprend un système anti-dévissage ayant des arêtes saillantes, ces arêtes constituent des zones très difficiles à isoler.

Enfin, un revêtement en parylène présente l'inconvénient d'avoir un coefficient de frottement relativement faible ce qui peut être néfaste au système de fixation et par conséquent réduire son efficacité, voire entrainer un dévissage de la capsule.

La présente invention vise à proposer un dispositif autonome implantable qui permette d'éviter la création de champs électriques entre les deux électrodes.

Cet aspect est particulièrement critique, dans la mesure où la durée de vie des capsules doit être préservée, et il est primordial d'avoir une bonne communication entre la capsule et le dispositif externe, afin d'assurer un fonctionnement optimal de la capsule.

Le US 2012/0172892 A1 décrit une capsule autonome de stimulation cardiaque comprenant un corps tubulaire avec une partie frontale munie de barbes d'ancrage et d'une électrode axiale. Ces éléments métalliques sont isolés du corps tubulaire par un assemblage complexe de quatre éléments en matériau isolant formant la partie frontale de la capsule en prolongement du corps tubulaire. Ces éléments sont configurés et agencés entre notamment pour permettre le passage des barbes d'ancrage au travers de la partie frontale, tout en entourant l'électrode axiale. Cette structure présente le double inconvénient d'être complexe (multiplicité des éléments et formes difficiles à réaliser) et délicate à assembler, ce qui du point de vue industriel grève le coût final du dispositif.

Les US 2012/172690 A1, US 2012/116489 A1 et US 2014/330326 A1 décrivent des capsules de type comparable, munies de moyens pour isoler la région frontale de la capsule du corps tubulaire.

Plus précisément, l'invention propose à cet effet une capsule implantable, notamment une capsule autonome de stimulation cardiaque, du type divulgué par le US 2012/0172892 A1 précité, c'est-à-dire comprenant un corps tubulaire abritant un ensemble d'éléments fonctionnels de la capsule et comprenant une électrode proximale, un élément distal pourvu à son extrémité distale d'un moyen d'ancrage à vis apte à pénétrer dans un tissu d'une paroi d'un organe d'un patient et d'une électrode distale, le corps, et des moyens d'isolation électrique entre le corps et l'élément distal pour isoler l'élément distal dudit corps.

De façon caractéristique, l'invention propose une capsule implantable selon la revendication 1.

Selon diverses caractéristiques subsidiaires avantageuses :
- ladite unique pièce en matériau isolant est une pièce moulée monobloc ;
- le moyen d'ancrage à vis est solidarisé à l'élément d'isolation électrique ;
- l'électrode distale est solidarisée à l'élément d'isolation électrique, l'élément d'isolation électrique comprenant un orifice traversant afin de relier électriquement l'électrode distale à une connexion électrique du corps ;
- l'électrode distale et la connexion électrique du corps sont reliées par un élément conducteur, l'électrode est fixée audit élément conducteur par tir laser ;
- l'élément d'isolation électrique présente un orifice à travers lequel est réalisé le tir laser ;
- le corps comprend à son extrémité distale, un support d'élément distal, ledit support d'élément distal comprenant un moyen de fixation de l'élément distal ;
- le support distal comprend un orifice pour l'accès à la connexion électrique du corps ;
- le moyen de fixation de l'élément distal comporte au moins un orifice et l'élément distal comprend au moins un plot apte à s'insérer dans l'orifice du moyen de fixation de l'élément distal ;
- l'élément d'isolation électrique comprend une surface d'appui transversal dans l'axe perpendiculaire à l'axe à distance de l'axe de la capsule et le support d'ancrage étant maintenu par un axe ou tige s'étendant dans un canal sous la surface d'appui transversal parallèle à la surface d'appui.

L'invention concerne également un procédé d'assemblage d'une capsule implantable tel que défini par la revendication 11.

On va maintenant décrire un exemple de mise en œuvre de la présente invention, en référence aux dessins annexés où les mêmes références désignent d'une figure à l'autre des éléments identiques ou fonctionnellement semblables.
La Figure 1 est une vue d'ensemble en perspective d'une capsule implantable selon l'invention.
La Figure 2 est une vue détaillée des différents éléments formant l'élément distal et le support de l'élément distal conformément à l'invention.
Les Figures 3a et 3b sont des vues des deux faces de l'élément d'isolation électrique selon un mode de réalisation.
La Figure 4 est une vue du support d'ancrage conformément à l'invention.
La Figure 5 est une vue de l'électrode distale.
Les Figures 6a et 6b sont des vues des deux faces du support d'élément distal.

On va maintenant décrire des exemples de réalisation d'une capsule implantable.

En référence tout d'abord à la Figure 1, il est représenté une capsule implantable 10, ici une capsule autonome de stimulation cardiaque, comprenant un corps tubulaire de capsule 12, un élément distal 14 pourvu à son extrémité distale d'un moyen d'ancrage 16 de type vis hélicoïdale et d'une électrode distale 18.

Le moyen d'ancrage 16 sous forme de vis est formé par un fil enroulé en hélice à pas de vissage à droite et est monté sur un support d'ancrage 20 intégrant des aménagements assurant l'irréversibilité de l'ancrage. Le moyen d'ancrage 16 est apte à pénétrer dans un tissu d'une paroi d'un organe d'un patient.

La vis 16 est rendue définitivement solidaire d'un support d'ancrage 20. On notera que les matériaux de la vis 16 et de son support d'ancrage 20 peuvent être les mêmes, par exemple de l'acier inoxydable de type 316L pour la vis 16 et pour le support d'ancrage 20.

De manière alternative, les matériaux de la vis et de son support d'ancrage peuvent être différents par exemple un couple platine/iridium 90/10 pour la vis et du titane pour le support d'ancrage.

Le corps de la capsule 12 abrite quant à lui, un ensemble d'éléments fonctionnels de la capsule, notamment un module électronique, une batterie et comprend également une électrode proximale 22.

Conformément à l'invention, la capsule comprend un élément d'isolation électrique 24 entre le corps de capsule 12 et l'élément distal 14 pour isoler électriquement l'élément distal 14 dudit corps 12.

De la sorte, l'élément distal 14, notamment l'électrode 18 et le moyen d'ancrage 16, est rendu flottant électriquement du corps de la capsule 12. La création de dipôle est donc évitée.

De façon caractéristique, l'élément d'isolation électrique 24 est un élément autonome, distinct du corps de capsule 12 et de l'élément distal 14, et se présentant sous forme d'une pièce monobloc unique.

Selon encore un autre mode de réalisation, le corps de capsule 12 comprend l'élément d'isolation 24.

L'élément d'isolation électrique 24 en raison de sa proximité avec la paroi cardiaque implique que le matériau de construction de cet élément soit en matériau biocompatible, notamment en plastique. Le matériau est par exemple du PEEK (polyétheréthercétone), matériau utilisé notamment pour les éléments de prothèses chirurgicales.

En outre, l'élément d'isolation électrique 24 doit être résistant afin d'être apte à résister aux mouvements de l'implant cardiaque et aux accélérations du cœur.

La Figure 2 illustre l'élément distal 14 ainsi que le support d'accroche 26 de cet élément distal sur le corps 12.

L'élément distal 14 de la capsule comprend notamment un sous-ensemble d'ancrage 28 formé par exemple d'un moyen d'ancrage 16 et d'un support d'ancrage 20. Ce sous-ensemble d'ancrage 28 comprend des aménagements pour permettre l'irréversibilité de la fixation du moyen d'ancrage 16 sur la paroi d'accroche de la capsule. Ces aménagements comprennent des encoches ou évidements 20a formés dans le support d'ancrage 20.

Le sous-ensemble d'ancrage 28 constitué des pièces 16 et 20 présente un diamètre de préférence égal à celui du corps de l'implant, typiquement de 6 à 7 mm, et une longueur axiale légèrement inférieure, typiquement entre 4 et 6 mm. Le moyen d'ancrage 16 délimite un espace libre occupé en partie par l'électrode de stimulation distale 18.

Le sous-ensemble d'ancrage 28 est conçu pour fixer l'implant de façon stable et durable dans le temps grâce à un moyen d'ancrage 16, par exemple une vis hélicoïdale formant un ressort à spires évolutives et se terminant par une pointe apte à perforer l'endothélium et à pénétrer dans les tissus musculaires, de façon à plaquer la paroi cardiaque sur la face frontale généralement annulaire du support d'ancrage 20, sensiblement à la même position (en direction axiale) que la surface d'appui interne du système d'électrode.

Plus en détail, le support d'ancrage 20 présente sur ladite face frontale une série d'évidements 20a qui réalisent la fonction d'irréversibilité une fois en contact avec l'endothélium. Dans cette position, le système de fixation est indémontable : sous l'action de la vis 16 qui agit comme un ressort de traction réalisant un effort de retenue axial, la paroi cardiaque est plaquée contre la face 20c du support d'ancrage 20 et s'ancre localement dans les évidements par l'effet ressort précité.

La vis en hélice 16 est ici constituée d'un fil métallique d'un diamètre de l'ordre de 0,5 mm, avec un diamètre d'enroulement de typiquement 5 mm environ et de préférence identique ou inférieur à celui du corps 12 de la capsule. La vis comprend une base plane suivie par deux spires jointives et une spire finale s'étendant par exemple sur environ 1,5 tour avec un espace inter-spire du même ordre de grandeur que le diamètre du fil. L'extrémité libre de la vis 16 est affinée, dans le cas d'espèce par deux usinages dans des plans mutuellement orthogonaux, créant ainsi une pointe perforante mais non tranchante. Le but de cette pointe est de permettre de traverser facilement l'endothélium puis de pénétrer dans le muscle cardiaque tout en créant un minimum de lésions tissulaires. La vis 16 est ici réalisée en acier inoxydable 316L implantable et biocompatible ou toute autre matière équivalente, qui délivre une raideur d'environ 0,1N/mm (raideur linéaire du ressort, mesurée par traction ou compression à l'aide d'un dynamomètre sur une course de 1 mm).

Ceci donne à la vis une souplesse axiale qui lui confère un effet ressort, opérant un effort de traction assurant un contact ferme entre le support d'ancrage 20 et la paroi cardiaque. Ainsi lors de la pénétration de la vis 16 dans le muscle, la vis se déforme axialement jusqu'au contact du bord libre 20c du support d'ancrage 20 avec l'endothélium. L'effet ressort de la vis va alors comprimer axialement l'endothélium et le muscle entre les spires et créer un effet de coincement. De plus, la proximité immédiate des spires et leur effet de traction sur l'endothélium forcent l'entrée de ce dernier dans les encoches d'anti-dévissage 20a. Au cours de ce mouvement, l'électrode de stimulation 18 vient ainsi en contact avec les cellules excitables du tissu de la paroi cardiaque. L'électrode de stimulation 18 est selon un exemple de réalisation, une pièce cylindrique avec la face en contact du tissu cardiaque en forme de dôme ou plan.

Selon un mode de réalisation particulier, l'électrode de stimulation 18 est dépourvue d'arête vive. D'autres configurations de spires sont bien entendu possibles, mais il est avantageux de prévoir un espacement entre les spires qui augmente entre la base de la vis (où comme on l'a vu l'espacement peut être nul) et l'extrémité de l'électrode de stimulation. Ceci permet de favoriser l'effort de traction axial appliquant le support d'ancrage 20 contre la paroi cardiaque.

On comprend par ailleurs que l'électrode distale 18 est en contact fiable et continu avec les tissus comprimés grâce à l'effort axial de la vis 16 en direction du support d'ancrage 20.

Une telle configuration est particulièrement adaptée pour une stimulation basse énergie, avec une longueur de l'électrode distale de l'ordre de 1,2 mm et un diamètre de l'ordre de 0,4 mm, soit une surface active de l'ordre de 1 mm².

Afin de maintenir fermement le moyen d'ancrage 16 sur le support d'ancrage 20, ce dernier présente une configuration comprenant un ensemble d'orifices traversant radiaux 20b, ayant une section correspondant approximativement à celle de tiges ou axes 30 et par exemple circulaire, formés dans la paroi du support d'ancrage 20, ces orifices recevant des tiges ou axes homologues 30. Les orifices 20b sont formés dans le support d'ancrage 20 à la périphérie de l'embase de la vis 16. Dans le présent exemple, on a prévu deux orifices traversant 20b, espacés par exemple de 180°, et deux tiges ou axes correspondants 30. Les tiges ou axes sont réalisés notamment en inox, par exemple de l'inox 316L.

Les orifices 20b coïncident avec la partie de la vis à spires jointives. Les tiges ou les axes 30 réalisés en un même matériau que la vis 16, ou en un matériau soudable par laser au matériau de la vis, est alors inséré dans chacun de ces trous latéraux.

Dans le cas (préféré) d'un axe, l'alésage des axes débouche alors sur les spires de la vis. Un tir laser à travers ces trous permet alors une soudure directe axe/vis assurant les conditions requises d'une bonne soudure laser, à savoir : i) compatibilité des matériaux, ii) contact direct et iii) accès visuel pour le tir et le contrôle de qualité.

Selon l'invention, le sous-ensemble d'ancrage 28 est solidarisé à l'élément d'isolation électrique 24.

En particulier, le support d'ancrage 20 est centré sur l'élément d'isolation électrique 24.

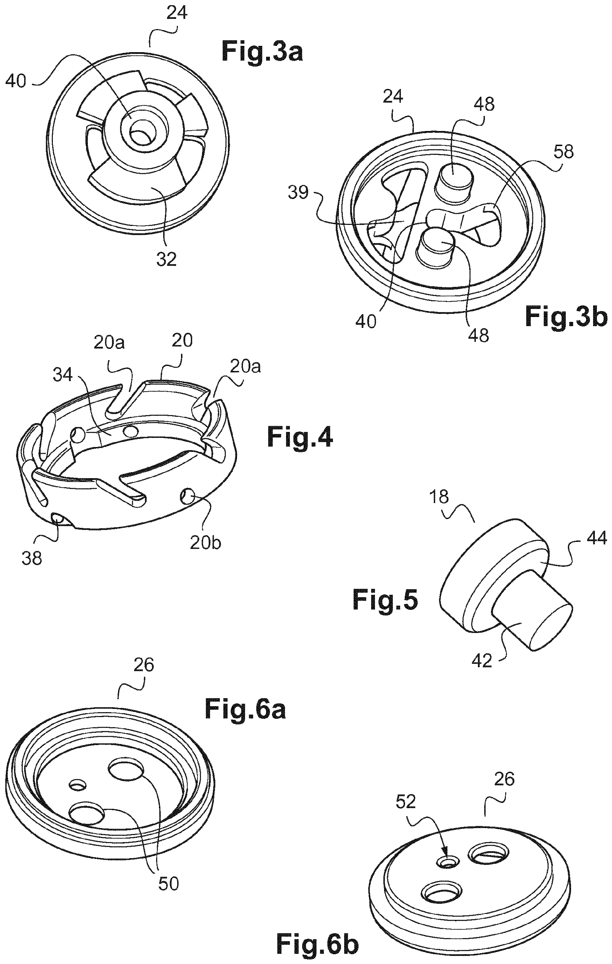

Selon un mode de réalisation, l'élément d'isolation électrique 24 comprend, comme montré en Figure 3a, une surface transversale 32 dans l'axe perpendiculaire à l'axe de la capsule ayant un diamètre donné, et servant de surface d'appui.

Selon un exemple de réalisation, le support d'ancrage 20 de forme cylindrique a un diamètre interne correspondant au diamètre externe de la surface transversale d'appui 32 afin de fixer le support d'ancrage 20 sur l'élément d'isolation électrique 24 en faisant glisser le support d'ancrage 20 à la périphérie extérieure de la surface transversale 32 de l'élément d'isolation électrique 24.

Selon un autre mode de réalisation, illustré en Figure 4, le support d'ancrage 20 comprend une lèvre intérieure 34 formée à la périphérie de l'embase du support d'ancrage au niveau de la zone de contact entre le support d'ancrage et l'élément d'isolation électrique 24, le diamètre interne de cette lèvre correspondant au diamètre externe de la surface transversale d'appui 32 de l'élément d'isolation électrique 24.

Selon ces modes de réalisation, il est ainsi réalisé un centrage par ajustement des deux pièces. En outre, il est aussi réalisé un blocage des deux pièces selon l'axe Y et selon l'axe Z tel que représenté en Figure 2.

Un blocage des deux pièces est également réalisé selon l'axe X par l'insertion d'une tige de blocage ou d'un axe de blocage 36 dans le support d'ancrage 20, notamment dans des orifices particuliers 38 représentés en Figure 2 et en Figure 4 formés à la périphérie de l'embase du support d'ancrage, sur la face latérale du support d'ancrage. La tige de blocage ou l'axe de blocage 36 est insérée d'une part dans l'un des orifices particuliers 38, puis traverse un canal 39 formé dans l'élément d'isolation électrique 24 tel que montré en Figure 2 et en Figure 3b puis traverse un second orifice particulier 38.

Le canal 39 est formé en particulier sous la surface d'appui 32 transversale parallèle à la surface d'appui et décalé par rapport à l'axe central de la capsule.

La tige de blocage ou l'axe de blocage 36 est fixé par soudure, par exemple par soudure laser aux deux extrémités de la tige sur le support d'ancrage 20.

Comme illustré en Figure 2 et en Figure 3a, l'élément d'isolation électrique 24 comprend un orifice traversant 40, par exemple positionné sensiblement au niveau de l'axe central de la capsule, permettant de fixer l'électrode distale 18 sur l'élément d'isolation électrique 24. Cet orifice est traversant afin de relier électriquement l'électrode distale 18 à une connexion électrique du corps de la capsule. L'électrode distale 18 illustrée en Figure 5 comprend une première partie 42 apte à s'insérer dans l'orifice traversant 40 de l'élément d'isolation électrique 24, la première partie 42 étant de forme complémentaire à l'orifice traversant 40 de l'élément d'isolation électrique 24.

Selon un exemple de réalisation, le trou traversant 40 et la première partie de l'électrode distale sont de forme cylindrique.

L'électrode distale 18 comprend une seconde partie 44 apte à venir en contact avec le tissu à stimuler. Selon un exemple particulier, la seconde partie de l'électrode distale présente une forme cylindrique de diamètre supérieur à la première partie, notamment afin de bloquer en profondeur l'insertion de l'électrode distale 18 dans l'orifice 40 de l'élément d'isolation électrique 24.

L'électrode distale 18 est par exemple fixée à l'élément d'isolation électrique 24 par collage, notamment au moyen d'une colle biocompatible. En effet, la colle étant susceptible de migrer à proximité du contour externe de la capsule, et donc d'être en contact avec la paroi cardiaque, la colle est par exemple de la colle époxy.

L'électrode distale 18 est réalisée par exemple en platine iridium et est enrobée d'un revêtement de type nitrure de titane (TiN).

L'installation de telle capsule doit permettre de préserver au maximum les tissus sur lesquels la capsule est implantée, afin de permettre par exemple une stimulation cardiaque efficace. Une telle implantation peut provoquer l'apparition d'une inflammation voir une nécrose des tissus et des cellules au niveau de l'emplacement de l'ancrage de la capsule et avoir pour conséquence une dégradation de l'efficacité des stimulations et donc la nécessité d'accroitre le seuil de stimulation.

Conformément à l'invention, une solution permettant de réduire de tels risques consiste à utiliser des stéroïdes en distalité des sondes cardiaques, notamment des capsules, de sorte que l'inflammation et la formation de fibrose soient diminuées.

Pour ce faire, l'élément distal comprend un anneau 46, par exemple un anneau en silicone, imprégné d'un produit stéroïde tel que la dexaméthasone.

Le produit stéroïde permet de réduire l'inflammation tissulaire durant les premières semaines qui suivent l'implantation.

Tel qu'illustré en Figure 2 et en Figure 3b, l'élément d'isolation électrique comprend sur la face opposée à la face sur laquelle est fixée le sous-ensemble d'ancrage 28 et l'électrode distale 18, au moins un plot 48, et de préférence deux plots.

Le corps de capsule 12 comprend à son extrémité distale, un support d'accrochage, aussi appelé support d'élément distal 26 illustré en Figure 2 et en Figures 6a et 6b. Le support d'élément distal 26 est réalisé par exemple en titane. Ce support d'élément distal 26 comprend au moins un moyen de fixation de l'élément distal 50.

Selon un mode de réalisation particulier, le moyen de fixation de l'élément distal comprend un ou plusieurs orifices 50, le ou les plots 48 de l'élément d'isolation électrique 24 étant aptes à s'insérer respectivement dans les orifices 50 du moyen de fixation de l'élément distal.

Le ou les plots 48 de l'élément d'isolation électrique 24 et les orifices 50 du support d'élément distal 26 sont de forme complémentaire et sont ajustés. Par exemple la forme des plots 48 et des orifices 50 est ronde, carré ou en forme d'étoile.

L'assemblage de l'élément d'isolation électrique 24 et du support d'élément distal 26 est tel qu'il bloque toute rotation entre ces deux pièces.

Selon un mode de réalisation, un verrouillage des deux pièces peut être réalisé. Par exemple, pour verrouiller la translation axiale du ou des plots 48, une opération de déformation de la matière des plots, par exemple des cylindres formant les plots, est réalisée pour bloquer ces plots dans leurs orifices respectifs de façon permanente et non démontable.

De manière complémentaire ou alternative, l'élément d'isolation électrique 24 et le support d'élément distal 26 peuvent être collés ensemble, notamment avec une colle biocompatible, par exemple de l'époxy. Un tel collage présente l'avantage de combler les interstices présents entre les deux pièces à l'assemblage et d'autre part de renforcer l'assemblage.

Le support d'élément distal 26 comprend aussi un orifice supplémentaire 52 relativement étroit, notamment de diamètre inférieur aux orifices d'accueil des plots de l'élément d'isolation électrique.

Le corps 12 de la capsule dans sa partie distale comprend un élément de fermeture 54 apte à fermer le corps de la capsule, notamment de manière étanche. Cet élément de fermeture 54 fixé au corps de la capsule comprend un orifice traversant 56 dans lequel est inséré un élément conducteur provenant de l'électronique interne de la capsule logée dans le corps 12. L'élément conducteur est par exemple un fil conducteur. L'électronique associée aux électrodes permet de mettre en œuvre, dans le cas d'une stimulation cardiaque, des fonctions de détection et de stimulation.

Le fil conducteur est guidé dans l'orifice supplémentaire 52 du support d'élément distal 26 pour ensuite être fixé sur l'électrode distale 18 du côté de la face de l'élément d'isolation électrique 24 portant un ou des plots 48 devant s'insérer dans le support d'élément distal 26.

Le fil conducteur comprend un isolant, par exemple une gaine thermorétractable de type PET (poly(téréphtalate d'éthylène)) afin d'éviter tout court-circuit.

L'élément d'isolation électrique 24 comprend un orifice ayant une forme de fenêtre 58 illustré en Figure 3b permettant de souder le fil électrique à l'extrémité de l'électrode distale 18, par exemple au moyen d'un tir laser. Selon un mode de réalisation l'orifice traversant 40 et l'orifice formant fenêtre 58 forment un même orifice.

Conformément à l'invention, le corps 12 de la capsule peut être recouvert d'un tube en polymère tel qu'un PET thermorétracté ou par un microrevêtement de type parylène C afin d'isoler le corps de la capsule. Cette isolation est par exemple positionnée sur le corps central en titane et s'étend de l'élément d'isolation électrique 24 jusqu'à environ 1 mm de la face proximale de la capsule pour laisser apparaitre un anneau d'environ 20 mm² constituant l'électrode proximale 22. Le dipôle ainsi constitué est distant d'une longueur environ de 25 mm. La longueur de l'électrode proximale peut variée de 1 à 5 mm et donc avoir une surface de 20 à 100 mm².

Selon l'invention, la capsule telle que précédemment décrite a une impédance d'environ 418 Ω et un rayonnement du champ électrique d'une valeur d'environ 159 mV/m.

Il va maintenant être décrit une solution d'assemblage de l'élément distal 14 et de son assemblage avec le support d'élément distal 26 et l'élément de fermeture 54 du corps 12 de la capsule.

Le problème que résout cette solution est de pouvoir rendre flottant électriquement le sous-ensemble d'ancrage 28 du reste de la capsule.

Les autres avantages sensibles de cette solution sont le faible encombrement et la très faible complexité de l'élément d'isolation électrique 24 à réaliser, notamment du fait que cet élément 24 est réalisé en matériau plastique. Un tel élément peut être réalisé par exemple par moulage par injection. L'élément d'isolation électrique 24 permet de réduire le rayonnement et l'efficacité du champ électrique créé entre l'électrode distale et l'électrode proximale.

Conformément à l'invention, le procédé d'assemblage de la capsule comprend les étapes suivantes de la revendication 11.

De façon détaillée, le procédé d'assemblage décrit ci-dessus comprend les sous-étapes suivantes :
1) Réalisation de l'assemblage du moyen d'ancrage 16 sur le support d'ancrage 20 par engagement du moyen d'ancrage à l'intérieur du support d'ancrage 20 et engagement d'un ensemble de tiges ou axes 30 dans les orifices 20b présents sur la périphérie du support d'ancrage 20, et soudage des tiges ou axes 30 sur le support d'ancrage 20, par exemple par tir laser,
2) Réalisation de l'assemblage du support d'élément distal 26 avec l'élément de fermeture 54 du corps de capsule, par engagement de l'élément de fermeture 54 du corps à l'intérieur du support d'élément distal 26. Cette étape est réalisée par exemple par estampillage et/ou par collage des deux pièces, par exemple avec de la colle polyuréthane et/ou un autre adhésif.
3) Montage de l'élément conducteur sur l'élément de fermeture 54 du corps de capsule, par insertion de l'élément conducteur dans l'orifice 52 prévu à cet effet et par soudure, notamment soudure laser de l'élément conducteur sur l'élément de fermeture 54 du corps de capsule.
4) Insertion de l'électrode distale 18 dans l'orifice 40 de l'élément d'isolation électrique 24, sur la face opposée à l'assemblage du support d'élément distal. L'électrode distale 18 est collée dans l'orifice 40 de l'élément d'isolation électrique 24, par exemple au moyen d'une colle polyuréthane ou d'une colle biocompatible de type époxy puis l'élément conducteur est fixé à l'électrode 18 par soudure, par exemple par tir laser appliqué au travers de l'orifice en forme de fenêtre 58 de l'élément d'isolation électrique 24.
5) Assemblage du support d'ancrage 20 muni d'un moyen d'ancrage 16 sur l'élément d'isolation électrique 24 par engagement de l'élément d'isolation électrique 24 dans le support d'ancrage 20, notamment par engagement de la surface transversale d'appui 32 de l'élément d'isolation électrique 24 à l'intérieur du support d'ancrage 20 et par soudage de l'axe de blocage 36 avec le support d'ancrage 20. Le blocage axial et la rotation du sous-ensemble d'ancrage 28 avec l'élément d'isolation électrique 24 sont effectués par l'axe de blocage 36 passant dans le canal 39.
6) Fixation de l'anneau 46 (collier stéroïdes) sur la surface transversale d'appui 32 au moyen d'une colle, par exemple colle silicone.

On notera que le "soudage" des étapes précédemment décrites ne doit pas être entendu au sens étroit d'une soudure mécanique avec fusion de la matière de deux pièces distinctes, mais d'une opération destinée à effondrer la matière et renforcer la fonction atraumatique par suppression des formes saillantes.

Les différents éléments et parties décrits ci-dessus peuvent être réalisés par usinage ou autre mise en forme selon des techniques conventionnelles.

La capsule peut être posée par le praticien selon la technique décrite notamment dans le document EP 2 394 695 A1, et extraite également selon une technique connue, la partie proximale 12 du corps de la capsule étant aménagée de façon adaptée.

## Revendications

1. Capsule implantable (10), notamment capsule autonome de stimulation cardiaque, comprenant :
- un corps tubulaire (12) abritant un ensemble d'éléments fonctionnels de la capsule et comprenant une électrode proximale ;
- un élément distal (14) pourvu à son extrémité distale d'un moyen d'ancrage à vis (16) apte à pénétrer dans un tissu d'une paroi d'un organe d'un patient et d'une électrode distale (18), le corps ; et
- des moyens d'isolation électrique entre le corps (12) et l'élément distal (14) pour isoler l'élément distal dudit corps, lesdits moyens d'isolation électrique comprenant un élément d'isolation électrique (24) constitué d'une unique pièce en matériau isolant (24) ;
**caractérisé en ce que**
le moyen d'ancrage à vis (16) est fixé sur un support d'ancrage (20) formant ainsi un sous-assemblage (28), le sous-assemblage (28) étant fixé à l'élément d'isolation électrique.

2. Capsule selon la revendication 1, **caractérisée en ce que** ladite unique pièce en matériau isolant (24) est une pièce moulée monobloc.

3. Capsule selon l'une quelconque des revendications précédentes, **caractérisée en ce que** le moyen d'ancrage à vis est solidarisé à l'élément d'isolation électrique.

4. Capsule selon l'une quelconque des revendications précédentes, **caractérisée en ce que** l'électrode distale est solidarisée à l'élément d'isolation électrique, l'élément d'isolation électrique comprenant un orifice traversant afin de relier électriquement l'électrode distale à une connexion électrique du corps.

5. Capsule selon la revendication 4, **caractérisé en ce que** l'électrode distale et la connexion électrique du corps sont reliées par un élément conducteur, l'électrode est fixée audit élément conducteur par tir laser.

6. Capsule selon la revendication 5, **caractérisé en ce que** l'élément d'isolation électrique présente un orifice à travers lequel est réalisé le tir laser.

7. Capsule selon l'une quelconque des revendications précédentes, **caractérisée en ce que** le corps comprend à son extrémité distale, un support d'élément distal (26), ledit support d'élément distal comprenant un moyen de fixation (50) de l'élément distal.

8. Capsule selon les revendications 5 et 7, **caractérisé en ce que** le support d'élément distal comprend un orifice pour l'accès à la connexion électrique du corps.

9. Capsule selon l'une des revendications 7 ou 8, **caractérisée en ce que** le moyen de fixation de l'élément distal comporte au moins un orifice et l'élément distal comprend au moins un plot apte à s'insérer dans l'orifice du moyen de fixation de l'élément distal.

10. Capsule selon la revendication 1, **caractérisée en ce que** l'élément d'isolation électrique comprend une surface d'appui transversal (32) dans l'axe perpendiculaire à l'axe à distance de l'axe de la capsule et le support d'ancrage étant maintenu par un axe ou tige s'étendant dans un canal sous la surface d'appui transversal parallèle à la surface d'appui.

11. Procédé d'assemblage d'une capsule implantable selon l'une des revendications 1-10, **caractérisé en ce que** le procédé comprend les étapes suivantes :
a) fixer le moyen d'ancrage à vis (16) sur le support d'ancrage (20) pour former le sous-assemblage (28);
b) fixer le sous-assemblage (28) sur l'
unique pièce en matériau isolant (24) formant l'élément d'isolation électrique entre le corps (12) et l'élément distal (14) ;
c) fixer l'électrode distale (18) sur ladite unique pièce en matériau isolant (24) ; et
d) fixer ladite unique pièce en matériau isolant (24) sur le corps tubulaire (12).

## Patentansprüche

1. Implantierbare Kapsel (10), insbesondere autonome Herzstimulationskapsel, bestehend aus:
- einem röhrenförmigen Körper (12), der eine Reihe von Funktionselementen der Kapsel aufnimmt und eine proximale Elektrode aufweist;
- einem distalen Element (14), das an seinem distalen Ende mit einem Schraubenverankerungsmittel (16), das in Gewebe einer Wand eines Organs eines Patienten eindringen kann, und einer distalen Elektrode (18) versehen ist; der Körper, und
- elektrischen Isoliermitteln zwischen dem Körper (12) und dem distalen Element (14) zum Isolieren des distalen Elements von dem Körper,
wobei die elektrischen Isoliermittel ein elektrisches Isolierelement (24) umfassen, das aus einem Einzelstück Isoliermaterial (24) besteht; **dadurch gekennzeichnet, dass** das Schraubenverankerungsmittel (16) an einem Verankerungshalter (20) befestigt ist, wodurch eine Unterbaugruppe (28) gebildet wird, wobei die Unterbaugruppe (28) an dem elektrischen Isolierelement befestigt ist.

2. Kapsel nach Anspruch 1, **dadurch gekennzeichnet, dass** das Einzelstück Isoliermaterial (24) ein einstückiges Formteil ist.

3. Kapsel nach einem der vorhergehenden Ansprüche, **dadurch gekennzeichnet, dass** das Schraubenverankerungsmittel fest mit dem elektrischen Isolierelement verbunden ist.

4. Kapsel nach einem der vorhergehenden Ansprüche, **dadurch gekennzeichnet, dass** die distale Elektrode fest mit dem elektrischen Isolierelement verbunden ist, wobei das elektrische Isolierelement eine Durchgangsöffnung aufweist, um die distale Elektrode mit einem elektrischen Anschluss des Körpers elektrisch zu verbinden.

5. Kapsel nach Anspruch 4, **dadurch gekennzeichnet, dass** die distale Elektrode und der elektrische Anschluss des Körpers über ein leitendes Element miteinander verbunden sind, wobei die Elektrode durch Laserschuss an dem leitenden Element befestigt ist.

6. Kapsel nach Anspruch 5, **dadurch gekennzeichnet, dass** das elektrische Isolierelement eine Öffnung aufweist, durch die der Laserschuss durchgeführt wird.

7. Kapsel nach einem der vorhergehenden Ansprüche, **dadurch gekennzeichnet, dass** der Körper an seinem distalen Ende einen Halter für das distale Element (26) aufweist, wobei dieser Halter ein Mittel (50) zur Befestigung des distalen Elements umfasst.

8. Kapsel nach Anspruch 5 und 7, **dadurch gekennzeichnet dass** der Halter für das distale Element eine Öffnung aufweist, die Zugang zum elektrischen Anschluss des Körpers bietet.

9. Kapsel nach Anspruch 7 oder 8, **dadurch gekennzeichnet, dass** das Mittel zur Befestigung des distalen Elements mindestens eine Öffnung und das distale Element mindestens einen Zapfen aufweist, der in die Öffnung des Mittels zur Befestigung des distalen Elements eingreifen kann.

10. Kapsel nach Anspruch 1, **dadurch gekennzeichnet, dass** das elektrische Isolierelement eine transversale Auflagefläche (32) in der Achse senkrecht zur Achse in einem Abstand zur Achse der Kapsel aufweist, wobei der Verankerungshalter durch einen Bolzen oder Stift gehalten wird, der sich in einem Kanal unterhalb der transversalen Auflagefläche parallel zur Auflagefläche befindet.

11. Verfahren zum Zusammenbau einer implantierbaren Kapsel nach einem der Ansprüche 1 bis 10, **dadurch gekennzeichnet, dass** das Verfahren die folgenden Schritte umfasst:
a) Befestigung des Schraubenverankerungsmittels (16) am Verankerungshalter (20) zur Ausbildung der Unterbaugruppe (28);
b) Befestigung der Unterbaugruppe (28) an dem Einzelstück Isoliermaterial (24), welches das elektrische Isolierelement zwischen dem Körper (12) und dem distalen Element (14) bildet;
c) Befestigung der distalen Elektrode (18) an dem Einzelstück Isoliermaterial (24); und
d) Befestigung des Einzelstücks Isoliermaterial (24) an dem röhrenförmigen Körper (12).

## Claims

1. An implantable capsule (10), in particular a cardiac stimulation autonomous capsule, said implantable capsule comprising:
- a tubular body (12) housing a set of functional elements of the capsule and provided with a proximal electrode;
- a distal element (14) provided at its distal end with anchor screw means (16) suitable for penetrating into tissue of a wall of an organ of a patient, and with a distal electrode (18), the body, and
- electrical insulation means for providing electrical insulation between the body (12) and the distal element (14) for insulating the distal element from said body; **characterized in that** said electrical insulation means comprising an electrical insulation element (24) made in one piece and of an insulating material (24);
**characterized in that**
the anchor screw means (16) are fastened to an anchor support (20), thereby forming a subassembly (28), the subassembly (28) being fastened to the electrical insulation element.

2. A capsule according to claim 1, **characterized in that** said one piece of insulating material (24) is an integrally molded piece.

3. Said capsule according to any preceding claim, **characterized in that** the anchor screw means are secured to the electrical insulation element.

4. A capsule according to any preceding claim, **characterized in that** the distal electrode is secured to the electrical insulation element, the electrical insulation element being provided with a through orifice so as to connect the distal electrode electrically to an electrical connection of the body.

5. A capsule according to claim 4, **characterized in that** the distal electrode and the electrical connection of the body are interconnected by a conductive element, and the electrode is fastened to said conductive element by firing a laser beam to perform laser beam welding.

6. A capsule according to claim 5, **characterized in that** the electrical insulation element is provided with an orifice through which the laser beam is fired to perform the laser beam welding.

7. A capsule according to any preceding claim, **characterized in that**, at its distal end, the body is provided with a distal element support (26), said distal element support being provided with fastening means (50) for fastening the distal element.

8. A capsule according to claims 5 and 7, **characterized in that** the distal element support is provided with an orifice for access to the electrical connection of the body.

9. A capsule according to claim 7 or claim 8, **characterized in that** the fastening means for fastening the distal element comprise at least one orifice, and the distal element is provided with at least one stud suitable for being inserted into the orifice in the fastening means for fastening the distal element.

10. A capsule according to claim 1, **characterized in that** the electrical insulation element has a transverse bearing surface (32) in the axis perpendicular to the axis remote from the axis of the capsule, and the anchor support being held by a pin or rod extending in a channel under the transverse bearing surface and parallel to the bearing surface.

11. A method of assembling an implantable capsule according to any one of claims 1 to 10, **characterized in that** the method comprises the following steps:
a) fastening the anchor screw means (16) to the anchor support (20) to form the subassembly (28);
b) fastening the subassembly (28) to the one piece of insulating material (24) forming the electrical insulation element between the body (12) and the distal element (14);
c) fastening the distal electrode (18) to said one piece of insulating material (24); and
d) fastening said one piece of insulating material (24) to the tubular body (12).
